# EUROPEAN PATENT SPECIFICATION

(11) **EP 0 426 815 B1**
(45) Date of publication and mention of the grant of the patent: **14.12.1994**
(21) Application number: 90908215.8
(22) Date of filing: 25.05.1990
(51) Int. Cl.: C09K 19/30, C07C 43/174, C07C 43/192, C07C 43/225, C07C 331/28, C07C 22/08, C07C 25/18

(54) **FLUORINATED BIPHENYL DERIVATIVES**
FLUORIERTE BIPHENYLDERIVATE
DERIVES DE BYPHENYLES FLUORURES

(30) Priority: 30.05.1989 GB 8912339
(43) Date of publication of application: 15.05.1991
(73) Proprietor: MERCK PATENT GmbH, D-64271 Darmstadt (DE)
(72) Inventor: COATES, David, Dorset BH21 3SW (GB); GREENFIELD, Simon, Dorset BH17 7YA (GB); SMITH, Graham, Dorset BH17 8AX (GB); CHAMBERS, Michael, Kevin, Dorset (GB); KURMEIER, Hans-Adolf, D-6104 Seeheim-Jugenheim (DE); DORSCH, Dieter, D-6105 Ober-Ramstadt (DE)
(86) International application number: EP9000847
(87) International publication number: WO9015115

(56) References cited:
- EP-A- 0 022 183
- EP-A- 0 051 738
- EP-A- 0 227 004
- EP-A- 0 256 636
- GB-A- 2 134 110

## Description

The invention relates to fluorinated biphenyl derivatives of formula I 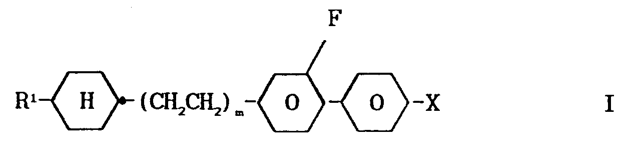
wherein
- R¹: denotes an alkyl residue with up to 12 carbon atoms wherein one or two non-adjacent CH₂-groups may also be replaced by -O-, -O-CO-, -CO-O- and/or -CH=CH-,
- m: is 0 or 1, and
- X: is -OCF₃, -OCHF₂
and also to liquid crystalline media being a mixture of at least 2 compounds, characterized in that at least one compound is a fluorinated biphenyl derivative according to formula I.

The invention was based on the object of discovering new stable liquid crystal or mesogenic compounds which are suitable as components of liquid crystalline media and, in particular, have advantageous values for optical and dielectric anisotropy combined with low viscosity and high nematogenity.

3,4-Difluorobiphenyls exhibiting a comparatively low viscosity are described in the USP 4797228. These biphenyls, however, have an unfavourable tendency to induce smectic phases especially at low temperatures.

EP-A-0 256 636 also teaches similar 3,4-difluorobiphenyls which have, however, a rather narrow nematic mesophase range. EP-A- 0 022 183 teaches similar cyclohexylbiphenyls without lateral fluorination.

It has now been found that fluorinated biphenyls of formula I are highly suitable as polar components of liquid crystalline media. In particular, they have especially advantageous values of optical and dielectric anisotropy and are not as smectogenic as 3,4-disubstituted analogues. It is also possible to obtain stable liquid crystal phases with a broad nematic mesophase range including a good deep temperature behaviour and a comparatively low viscosity with the aid of these compounds.

Depending on the choice of the substituents, the compounds of the formula I can be used as the base materials from which liquid crystal media are predominantly composed; however, it is also possible for compounds of the formula I to be added to liquid crystal base materials of other classes of compounds, for example in order to influence the dielectric and/or optical anisotropy and/or the viscosity and/or the nematic mesophase range of such a dielectric.

The compounds of the formula I are colourless in the pure state and are liquid crystalline in a temperature range which is favourably placed for electrooptical use. They are very stable towards chemicals, heat and light and allow the realization of liquid crystalline media which have significantly improved electrical resistivity values and in particular show an improved resistivity behaviour under thermal and/or UV-load.

The invention thus relates to the fluorinated biphenyl derivatives of the formula I, to liquid crystalline media with at least two liquid crystalline components, wherein at least on component is a compound of the formula I and to liquid crystal display devices containing such media.

Above and below, R¹, m and X have the meaning given unless expressly indicated otherwise.

The compounds of the formula I include cyclohexyl biphenyl derivatives of the formulae I1 and I2: 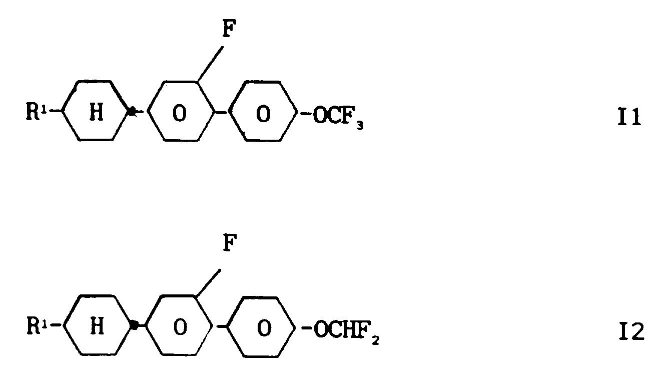
and cyclohexylethyl biphenyl derivatives of the formulae I3 and I4; 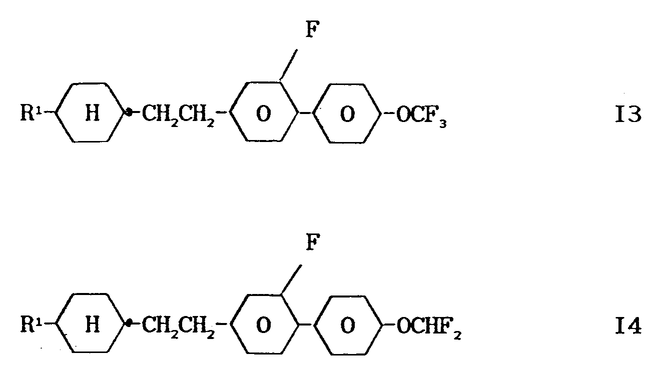
R¹ is preferably alkyl, alkoxy, oxaalkyl, alkanoyloxy or alkenyl and can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2-(= ethoxymethyl) or 3-oxybutyl (= 2-methoxyethyl), 2-, 3- or 4-oxapentyl, 2-, 3-, 4- or 5-oxahexyl, 2-, 3-, 4- 5- or 6-oxaheptyl, 2-, 3-, 4-, 5-, 6-or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 or 10 C atoms. It is accordingly, in particular, vinyl, prop-1- or prop-2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be of importance because of an improved solubility in the customary liquid crystal base materials, but in particular as chiral doping substances if they are optically active.

Branched groups of this type as a rule contain not more than one chain branching. Preferred branched radicals are iso propyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 4-methylheptyloxycarbenyl, 2-methylbutyryloxy, 3-methylvaleryloxy, 4-methylhexanoyloxy, 2-methyl-3-oxapentyl and 2-methyl-3-oxahexyl.

In the case of compounds with a branched terminal group R¹, formula I includes both the optical antipodes and racemates as well as mixtures thereof.

Of the compounds of the formula I and subformulae thereof, those in which at least one of the radicals contained therein has one of the preferred meanings given are preferred.

The compounds of the formula I are prepared by methods which are known per se, such as are described in the literature (for example in the standard works, such as Houben-Weyl, Methoden der Organischen Chemie Methods of Organic Chemistry, Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formula I.

A preferred route for preparation of the compounds of the formula I is shown in the following scheme 1: 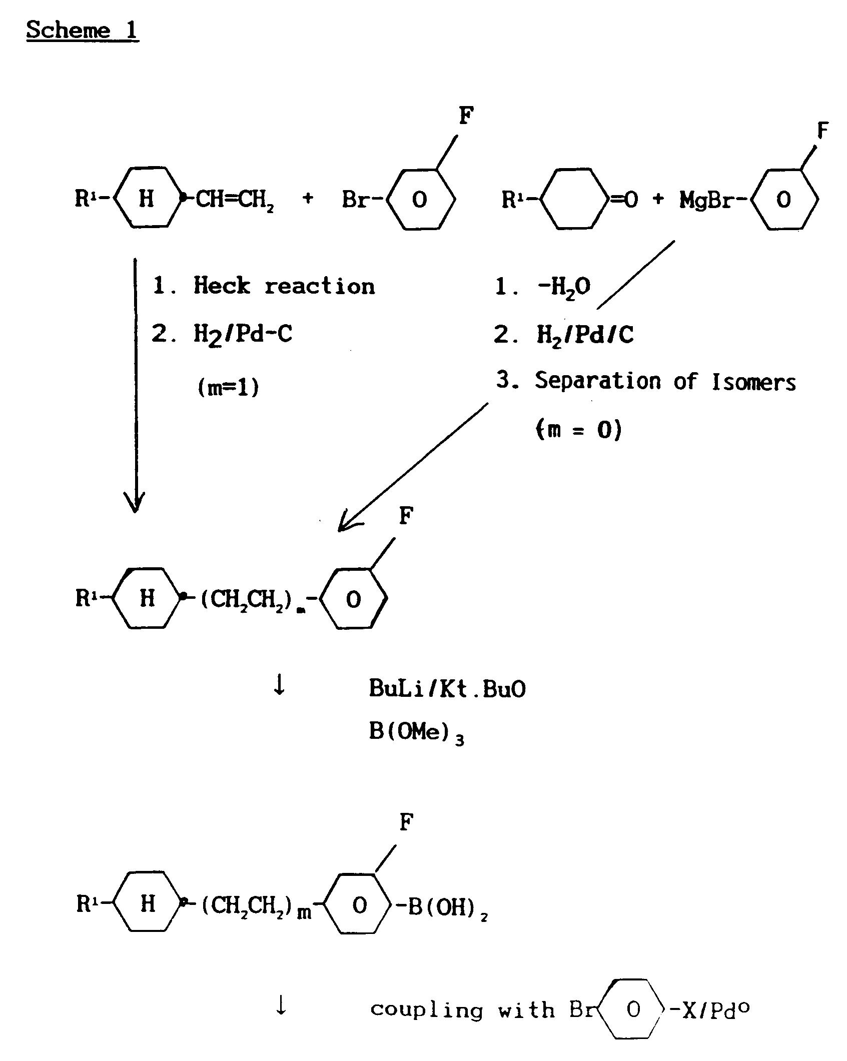 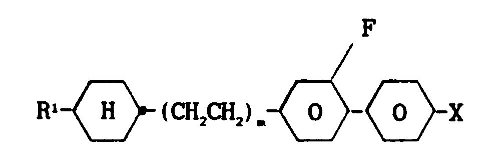
An alternative route is shown in scheme 2: 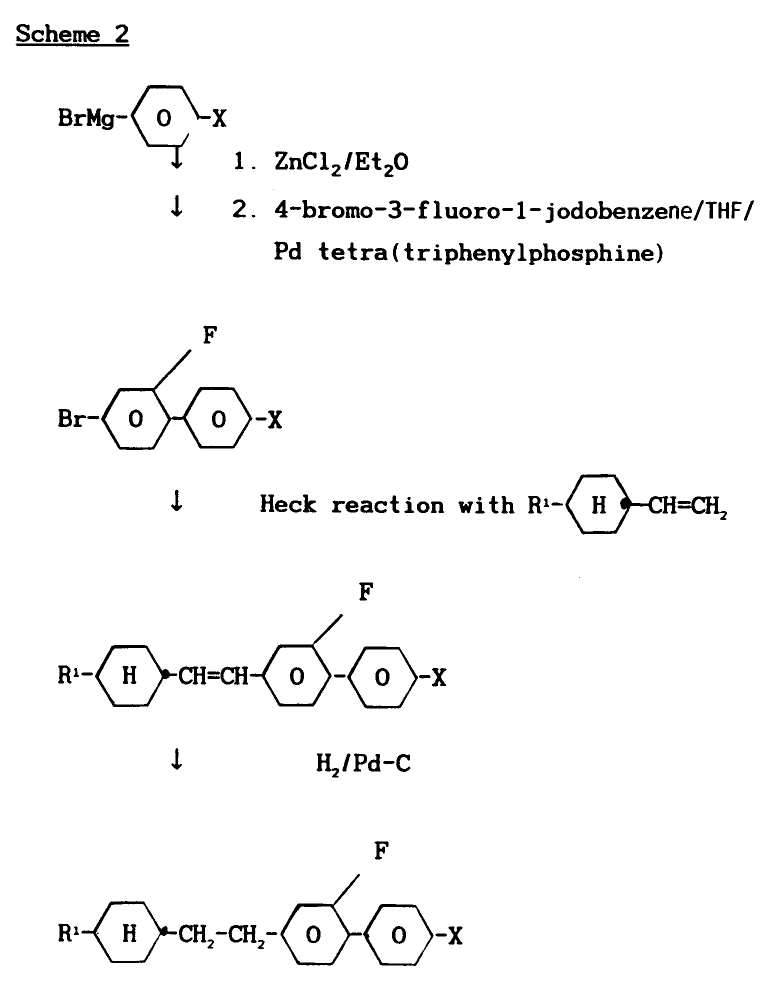
Another route is shown in the following scheme 3: 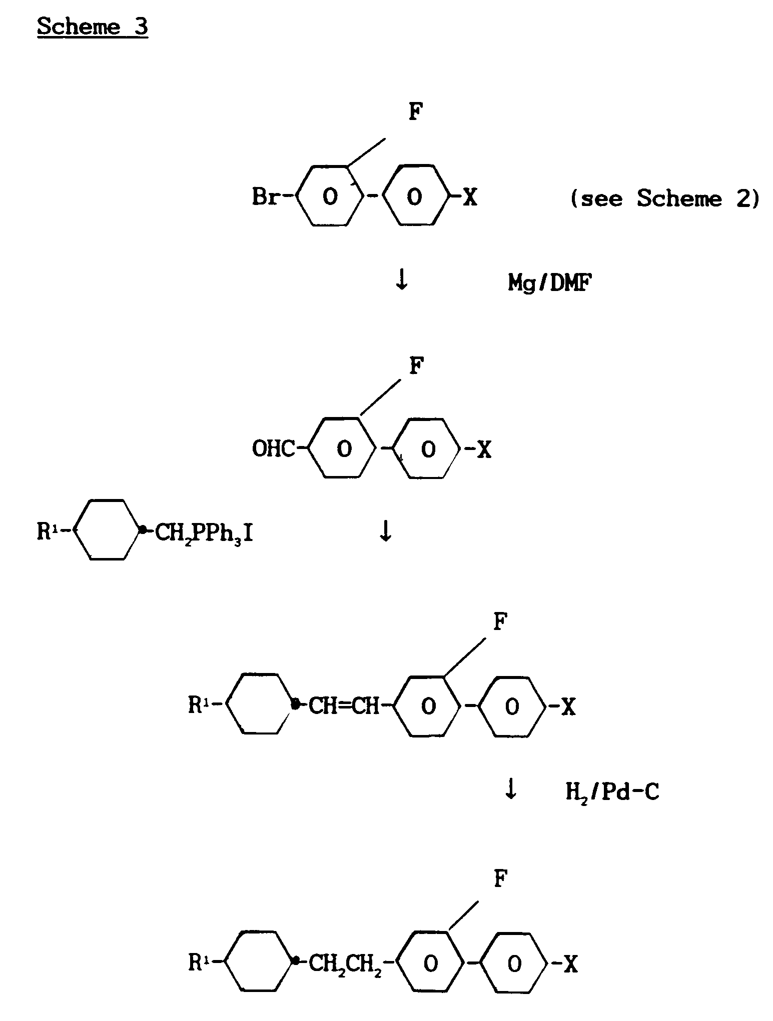
A preferred route for preparation of the compounds of formula I1 is shown in the following scheme 4: 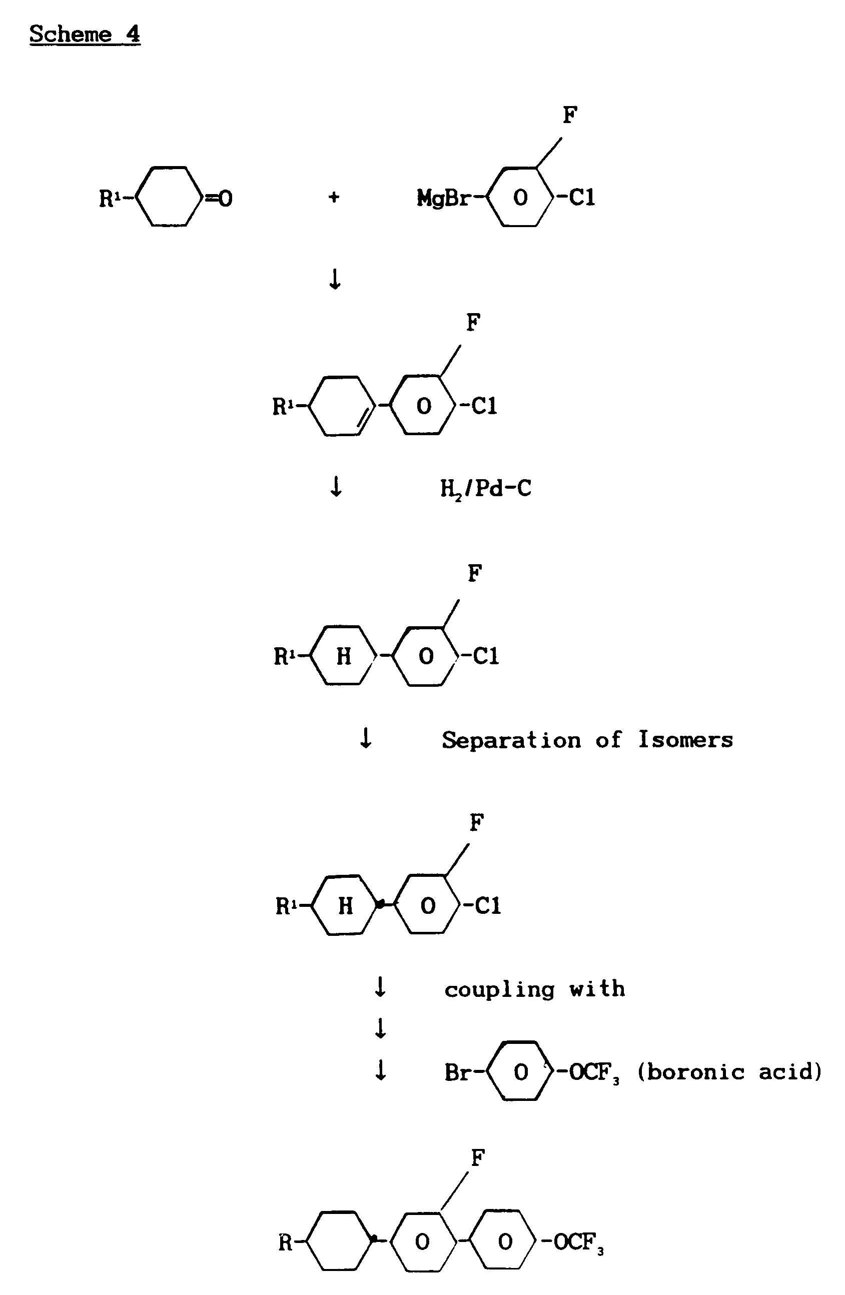
Finally preferred routes for preparation of the compounds of formula I1 or I2 are shown in the following schemes 5 and 6: 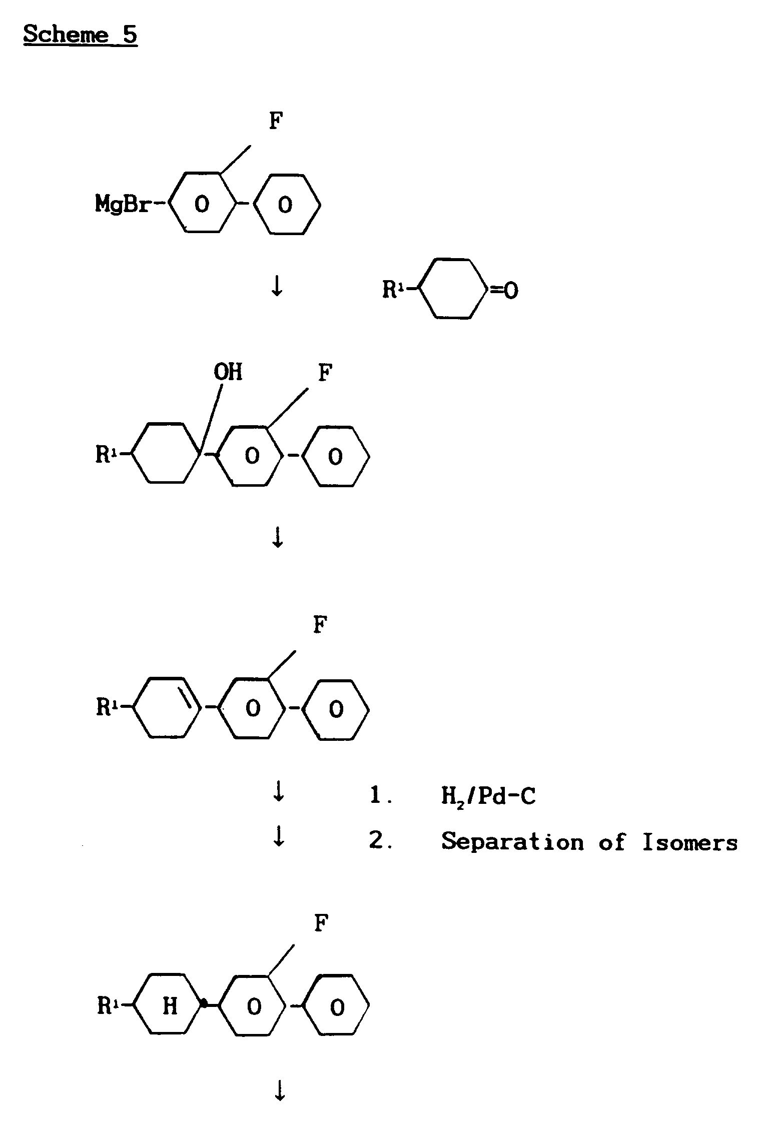 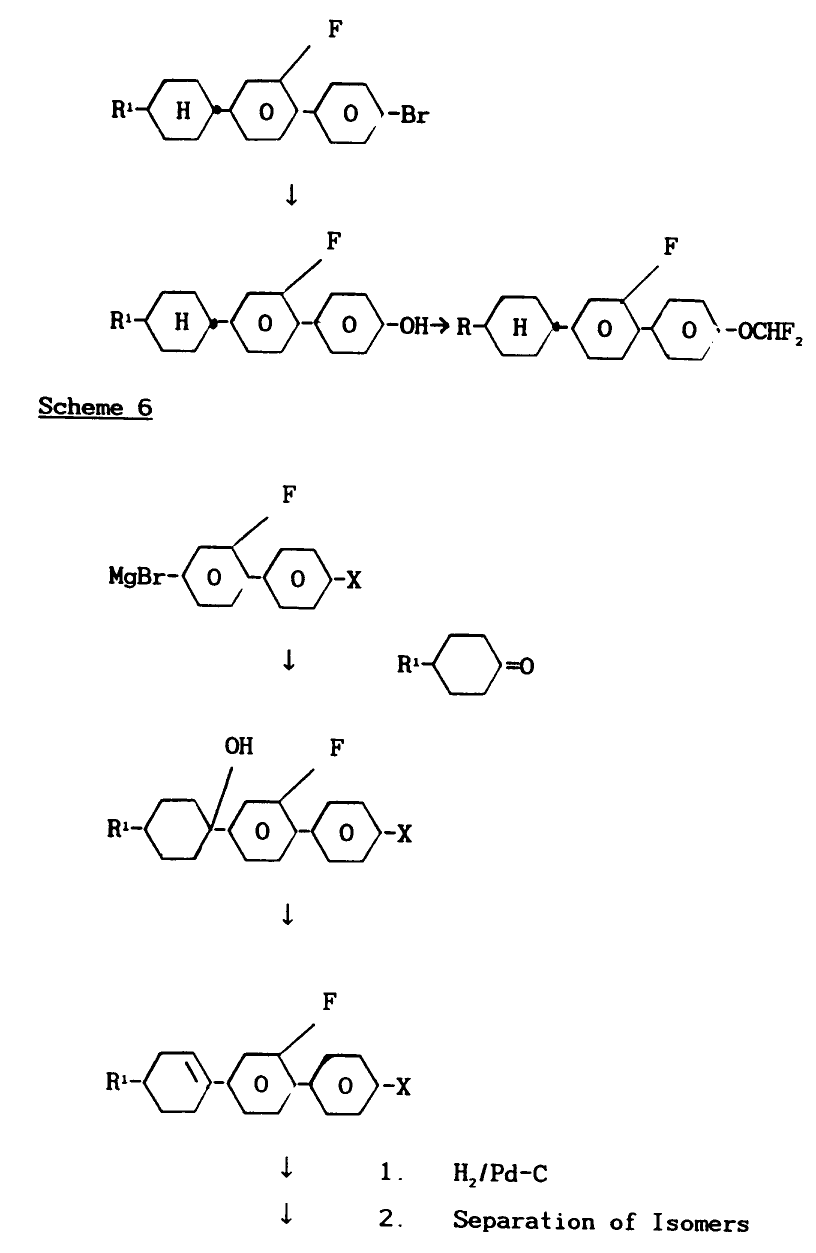 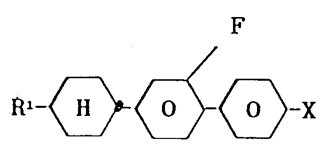
X = OCF₃ or OCHF₂
Other routes are apparent to the skilled worker. In addition it is possible to follow the above shown routes with a group X being different from the desired one and to introduce the desired group X in the final step, e.g. conversion of -OMe to -OH and finally to -OCF₃ or OCHF₂. All these steps and the corresponding reaction conditions are known to the skilled worker.

The starting materials are known or can be prepared in analogy to known compounds. The 2-fluoro-4-bromo-4'-X-biphenyls (X = OCF₃ or OCHF₂), for example, can be made by transition metal catalyzed cross-coupling reactions (E. Poetsch, Kontakte (Darmstadt) 1988 (2) p. 15): 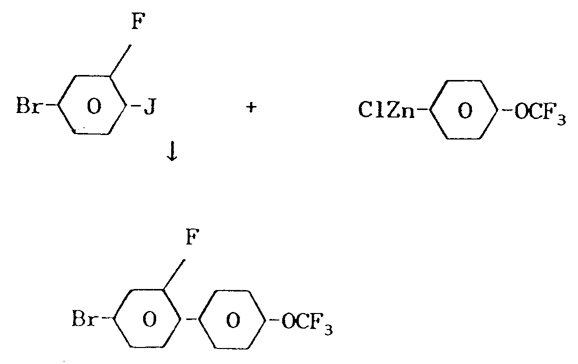
In addition to one or more compounds for formula I the liquid crystal media according to the invention preferably contain 2-40 components and in particular 4-30 components. Liquid crystal media being composed of one or more compounds of formula I and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates_{,} cyclohexylphenylbenzoates, cyclohexylphenyl cyclohexanecarboxylates, cyclohexylphenyl cyclohexylcyclohexanecarboxylates_{,} phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls_{,} phenyl- or cyclohexylpyrimidines, phenyl- or cyclohexylpyridines_{,} phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2⁻(4-phenyl-cyclohexyl)-ethanes, 1-cyclohexyl-2-biphenylethanes_{,} 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal media according to the invention can be characterized by the formalae 1, 2, 3, 4 and 5:

R'-L-U-R'' 1

R'-L-COO-U-R'' 2

R'-L-OOC-U-R'' 3

R'-L-CH₂CH₂-U-R'' 4

R'-L-CC-U-R'' 5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans- 1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one ore more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R'' are independently from each other alkyl, alkenyl, alkoxy, alkenoxy with up to 8 carbon atoms. R' and R'' differ from one another in most of these compounds, one of the residues usually being alkyl or alkenyl. In another preferred subgroup of the compounds of formulae 1, 2, 3, 4 and 5 (subgroup 2) R'' denotes -CN, -CF₃, -F, -Cl or -NCS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal media according to the invention preferably contain in addition to components selected from subgroup 1 also components of subgroup 2, the percentage of these components being as follows:
- subgroup 1:: 20 to 90 %, in particular 30 to 90 %
- subgroup 2:: 10 to 50 %, in particular 10 to 50 %
In these liquid crystal media the percentages of the compounds according to the invention and the compounds of sub group 1 and 2 may add up to give 100 %.

The media according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/ R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

The following examples are to be construed as merely illu strative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and/or chromatography.

Further are:
C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

### Examples for production

### Comparative Example

A mixture of 0.1 mole of 1-chloro-2-fluoro-4-(trans-4-n-propylcyclohexylethyl)benzene (obtained by the reaction of 4-n-propylcyclohexylmethylphosphonium iodide with 4-chloro-3-fluorobenzaldehyde followed by hydrogenation), 0.1M 4-fluorophenylboronic acid (obtained by the reaction of 4-bromofluorobenzene with magnesium and trimethylborate, tetrakis(triphenylphosphine)palladium (1 mole %) 2m sodium carbonate solution (150 ml), toluene (250 ml) and IMS (60 ml) is stirred and refluxed for 16 hrs under a nitrogen atmosphere. After cooling the organic layer is separated, washed with water and the solvent evaporated off. The crude 4-(trans-4-propylcyclohexylethyl)-2,4'-difluorobiphenyl is purified by chromatography on silica and crystallisation, C 47 N 89 I.

The following compounds are obtained analogously:

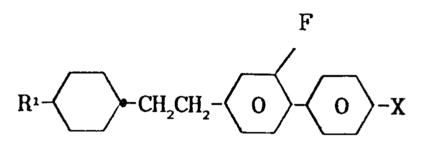

| R¹ | X | |
|---|---|---|
| n-propyl | OCF₃ | C 36 N 89.1 I |
| n-pentyl | OCF₃ | |
| n-propyl | OCHF₂ | |
| n-pentyl | OCHF₂ | |

### Example 1

A mixture of 0.1m 4-(trans-4-propylcyclohexyl)-2-fluoro-1-chlorobenzene (obtained by the reaction of 4-propylcyclohexanone and 3-4-chlorophenylmagnesium bromide), 0.1m trifluoromethoxyphenylboronic acid, tetrakis (triphenyl phosphine)palladium (1 mole %), 2m sodium carbonate (150 ml), toluene (250 mls) and IMS (60 ml) is stirred for 16 hrs under reflux in a nitrogen atmosphere. After the usual work up and purification by chromatography and crystallization the pure 4-(trans-4-propylcyclohexyl)-2-fluoro-4'-trifluoromethoxybiphenyle is obtained.

The following compounds are obtained analogously:

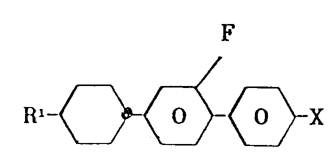

| R¹ | X | |
|---|---|---|
| n-pentyl | OCF₃ | C 56 N 107.4 I |
| n-nonyl | OCF₃ | |
| n-heptyl | OCF₃ | |
| n-butyl | OCF₃ | |
| ethyl | OCF₃ | |
| ethyl | OCHF₂ | |
| n-propyl | OCHF₂ | |
| n-butyl | OCHF₂ | |
| n-pentyl | OCHF₂ | |
| n-heptyl | OCHF₂ | |

### Example 2

### Step 1 and Step 2

Butyl lithium (1.6 m) (95 ml) was slowly added to trans 1,4-propylcyclohexylmethyl phosphonium iodide (70 g) in THF (140 ml) at 20 °C and stirred for 1 hr. 3-fluorobenzaldehyde (16.5 g) in THF (20 ml) was added dropwise and stirred for 15 mins. Water (100 ml) was added and the volatiles distilled off Dichloromethane (200 ml) was added and the organic layer separated, washed with dilute hydrogenperoxide and then ammonium ferrous sulphate and water. Column chromatography gave the cis/trans alkene which was hydrogenated with Pd/C in THF (100 ml) to give the required ethane (cf. Scheme 1).

### Step 3

Product from step 2 (17.8 g) dissolved in THF (30 ml), potassium butoxide (8 g) in THF (30 ml) and DMPU (9.5 g) were cooled to -110 °C. Butyl lithium (1.6 m) (50 ml) was added dropwise over 40 min and stirred at -100 °C for 1 hr. Trimethyl borate (8.2 g) in THF (20 ml) was added and the reaction mixture allowed to warm to 20 °C. Normal work up gave the required boronic acid.

### Step 4

A mixture of boronic acid (1.9 g) from the previous step, 4-bromotrifluoromethoxybenzene (1.6 g), toluene (20 ml), ethanol (5 ml), palladium tetra (triphenylphosphine) (0.1 g) and sodium carbonate solution (10 ml) was stirred and heated at reflux for 5 hrs. The product was isolated by extraction and column chromatography to give 4-(trans-4-n-propylcyclohexylethyl)-2-fluoro-4'-trifluoromethyoxybiphenyl:
C 36 N 89 I.

### Example 3

### Step 1

The Grignard reagent of 4-bromo trifluoromethoxy benzene (75.9 g) was prepared by reaction with magnesium (8.3 g) in THF (515 ml), after cooling to 20 °C, zinc chloride (315 ml of 1.0 m soln in diethyl ether) was added with cooling, excess magnesium was filtered off. This mixture was added to a mixture of 4-bromo-3-fluoro-1-iodobenzene (86.2 g) in THF (150 ml) containing palladium tetra (triphenylphosphine) (1.0 g) and stirred for 2 days under nitrogen. Normal workup and chromatography gave 4-bromo-2-fluoro-4'-trifluoromethoxybiphenyl.

### Step 2

The product from step 1 (18.6 g) was converted to the Grignard reagent using magnesium (1.4 g) and THF (70 ml). Trans 4-Pentylcyclohexanone (8.7 g) in THF (20 ml) was slowly added and the mixture then heated under reflux for 1 hr. After the usual workup 22.5 g of viscous oil was isolated, this was dehydrated using toluensulphonic acid (0.2 g) in toluene (100 ml) to give the alkene (Scheme 6). After hydrogenation of the double bond the trans-isomer was isolated by extraction and column chromatography to give 4-(trans-4-n-pentylcyclohexyl)-2-fluoro-4'-trifluoromethoxybiphenyl: C 56 N 107 I

Other end group such as OCHF₂ and all alkyl homologues including ethyl, n-propyl,, n-butyl, n-hexyl and n-heptyl can be made in the same way.

### Example 4 (Use Example)

A liquid crystalline medium consisting of 90 % of ZLI-3086 (commercially available from E. Merck, Darmstadt, FRG) and 10 % of 4-(trans-4-n-propylcyclohexylethyl)-2-fluoro-4'-trifluoromethoxybiphenyl exhibits a clearing point of 72.2 °C, a viscosity of 11.6 cSt, a birefringence Δ n of 0.113 and a dielectric anisotropy Δε of +0.8.

## Claims

1. Fluorinated biphenyl derivatives of formula I 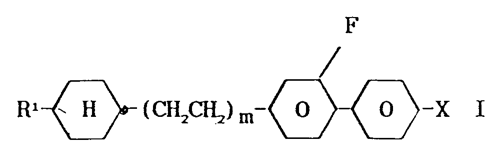 wherein
R¹ denotes an alkyl residue with up to 12 carbon atoms wherein one or two non-adjacent CH₂-groups may also be replaced by -O-, -O-CO-, -CO-O- and/or -CH=CH-,
m is 0 or 1, and
X is -OCF₃, -OCHF₂.

2. Biphenyl derivatives of Claim 1, wherein R¹ is straight-chain alkyl of 2, 3, 4, 5, 6 or 7 C atoms.

3. Liquid crystalline medium being a mixture of at least two compounds, characterized in that at least one compound is a fluorinated biphenyl derivative according to claim 1.

4. Liquid crystal display device, characterized in that it contains a liquid crystalline medium according to claim 3.

5. Electrooptical display device, characterized in that it contains a liquid crystalline medium according to claim 3.

## Patentansprüche

1. Fluorierte Biphenylderivate der Formel I 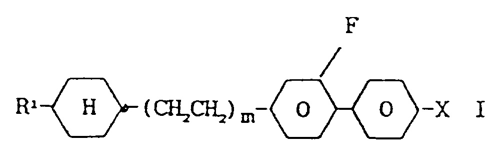 worin
R¹ einen Alkylrest mit bis zu 12 Kohlenstoffatomen, wobei eine oder zwei nicht benachbarte CH₂-Gruppen auch durch -O-, -O-CO-, -CO-O- und/oder -CH=CH-ersetzt sein können,
m 0 oder 1 und
X -OCF₃, -OCHF₂ bedeuten.

2. Biphenylderivate des Anspruchs 1, worin R¹ geradkettiges Alkyl mit 2, 3, 4, 5, 6 oder 7 C-Atomen bedeutet.

3. Flüssigkristallines Medium aus einer Mischung von mindestens zwei Verbindungen, dadurch gekennzeichnet, daß mindestens eine Verbindung ein fluoriertes Biphenylderivat nach Anspruch 1 darstellt.

4. Flüssigkristall-Anzeigevorrichtung, dadurch gekennzeichnet, daß sie ein flüssigkristallines Medium nach Anspruch 3 enthält.

5. Elektrooptische Anzeigevorrichtung, dadurch gekennzeichnet, daß sie ein flüssigkristallines Medium nach Anspruch 3 enthält.

## Revendications

1. Dérivés de biphényle fluorés de formule I 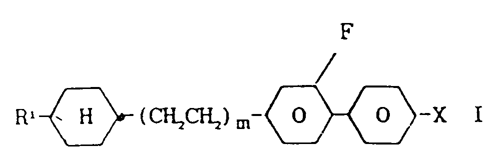 dans laquelle
R¹ dénote un résidu alkyle comportant jusqu'à 12 atomes de carbone où un ou deux groupes CH₂ non adjacents peuvent également être remplacés par -O- , -O-CO-, -CO-O- et/ou -CH=CH- ,
m vaut 0 ou 1, et
X représente -OCF₃ , -OCHF₂ .

2. Dérivés de biphényle de la revendication 1, dans lesquels R¹ est un alkyle à chaîne droite comportant 2, 3, 4, 5, 6 ou 7 atomes de carbone.

3. Milieu à cristaux liquides qui est un mélange d'au moins deux composés, caractérisé en ce qu'au moins un composé est un dérivé de biphényle fluoré selon la revendication 1.

4. Dispositif d'affichage à cristaux liquides, caractérisé en ce qu'il contient un milieu cristallin liquide selon la revendication 3.

5. Dispositif d'affichage électro-optique, caractérisé en ce qu'il contient un milieu cristallin liquide selon la revendication 3.
